# EUROPÄISCHE PATENTSCHRIFT

(11) **EP 0 387 688 B1**
(45) Veröffentlichungstag und Bekanntmachung des Hinweises auf die Patenterteilung: **08.12.1993**
(21) Anmeldenummer: 90104399.2
(22) Anmeldetag: 08.03.1990
(51) Int. Cl.: C07C 41/16, C07C 43/15

(54) **Verfahren zur Herstellung unsymmetrischer, endständig einfach ungesättigter Glykolether**
Method of producing asymmetrical glycol ethers with a terminal isolated double bond
Procédé pour la fabrication de glycol-éthers asymmétriques avec double liaison terminale isolée

(30) Priorität: 17.03.1989 DE 3908792
(43) Veröffentlichungstag der Anmeldung: 19.09.1990
(73) Patentinhaber: HÜLS AKTIENGESELLSCHAFT, 45764 Marl (DE)
(72) Erfinder: Rauleder, Hartwig, Dr., D-7888 Rheinfelden (DE); Kötzsch, Hans-Joachim, Dr., D-7888 Rheinfelden 1 (DE); Vahlensieck, Hans-Joachim, Dr., D-7867 Wehr (DE)

(56) Entgegenhaltungen:
- EP-A- 0 238 850
- DE-C- 714 490
- US-A- 2 841 621

## Beschreibung

Die Erfindung betrifft ein Verfahren zur Herstellung unsymmetrischer, endständig einfach ungesättigter Glykolether der Formel

R¹ - (O-R³) _{1 bis 6} - OR² (I),

worin R³ gleiche oder verschiedene Alkylengruppen von 2 bis 4 C-Atomen in der Kette mit ggf. an die C-Atome gebundenen Methylgruppen, R¹ einen geradkettigen oder verzweigten Alkylrest mit 1 bis 4 C-Atomen und R² einen endständig ungesättigten Alkenylrest mit 2 bis 6 C-Atomen in der Kette mit ggf. an die C-Atome gebundenen Methyl- oder Ethylgruppen bedeuten, durch Überführung von Monoalkylethern der Formel

R¹-(O-R³)_{1 bis 6} - OH (II),

mit Alkalimetallen bzw. Erdalkalimetallen, deren Alkoholaten bzw. Oxiden, Hydriden oder Hydroxiden in die Alkoholate und nachfolgender Umsetzung der Alkoholate mit einem endständig ungesättigten Alkenylhalogenid der Formel

X - R² (III),

worin X Chlorid, Bromid, oder Jodid bedeuten und R² die obengenannte Bedeutung besitzt.

Die Herstellung von reinen Produkten der Formel (I) in vetretbaren Ausbeuten nach der WILLIAMSON-Synthese scheiterte bisher an entstehenden azeotropen Gemischen von (I) mit den Ausgangsstoffen der Formel (II). Trotz hohen destillativen Aufwands, der von Nachteil ist, wurden stets geringe Mengen reines (I) neben großen Mengen von Gemischen aus (I) und (II) erhalten, die für die Weiterverarbeitung ungeeignet sind. Versuche, das Alkoholat vollständig ohne Verwendung eines Überschusses von (II) zu bilden und dem Entstehen des Azeotrops auszuweichen, blieben erfolglos. Sehr lange Reaktionszeiten führen darüber hinaus zu Nebenreaktionen und Ausbeuteverlusten. Der Ausgangsstoff (II) konnte nach dem Stand der Technik nicht vollständig zur Reaktion gebracht werden.

Diese Schwierigkeiten werden durch das erfindungsgemäße Verfahren in einfacher Weise überwunden. Entgegen dem bisherigen Kenntnisstand führt das erfindungsgemäße Verfahren in der Alkoholatstufe mit überschüssigem (II) schnell und schonend zur vollständigen Umsetzung in das Alkoholat und nach dessen Umsetzung mit (III) zu einem Gemisch aus (I) und (II), in dem das gesamte (II) - durch weitere zunächst nur teilweise Überführung in das Alkoholat - überraschenderweise so in nichtflüchtiger Form gebunden wird, daß das im Gemisch anwesende (I) als reines Produkt aus dem Reaktor abdestilliert werden kann. Das (II)-Alkoholat bleibt dabei intakt und ist nach Komplettierung der Bildung von (II)-Alkoholat durch Zusatz von weiterem (II) für die Herstellung von weiterem (I) geeignet.
Auf diese Weise werden die Produkte der Formel (I) in sehr hohen, nahezu quantitativen Ausbeuten als reines Produkt gewonnen, das in der Regel bereits so rein ist, daß es nicht mehr nachdestilliert werden muß.
Die Aufgabe, solche endständig ungesättigten, unsymmetrischen -α, ω Glykolbisether technisch in wirtschaftlicher Weise zugänglich zu machen, ergab sich aus deren Bedeutung als Ausgangsstoffe für die Hydrosilierung, d.h. Anlagerung von Hydrogensilanen. Diese hydrosilierten unsymmetrischen Glykolether sind Träger wichtiger Eigenschaften in Hydraulikflüssigkeiten.

Gegenstand der Erfindung ist ein Verfahren zur Herstellung der genannten unsymmetrischen Ether (I) aus den Alkoholaten der Monoalkylether (II) durch Umsetzung mit Alkenylhalogenide (III), dadurch gekennkzeichnet, daß
a) man zunächst zwei Äquivalente (II) mit einem Äquivalent der alkoholatbildenden Metallkomponente bis zu deren vollständigem Umsatz unter Sauerstoff-Ausschluß umsetzt, wobei die jeweilige Fluchtgruppe abdestilliert wird,
b) das entstandene (II)-Alkoholat mit einem Äquivalent (III) ausreagieren läßt,
c) das erhaltene Gemisch der Stoffe (I) und (II) vom ausgefällten Metallhalogenid abfiltriert oder vollständig abdestilliert,
d) das Destillat bzw. Filtrat mit einem zweiten Äquivalent des Alkoholat-Bildners unter den Bedingungen von a) umsetzt,
e) danach das Produkt (I) weitgehend abdestilliert und
f) dem nach der Gewinnung von (I) zurückbleibenden alkoholathaltigen Reaktionsgemisch ein weiteres Äquivalent (II) zusetzt, die Umsetzung gemäß a) zu Ende führt und wieder die Verfahrensschritte a) bis e) ausführt.

Das Verfahren hat die Besonderheit, daß häufig, zumindest mehrmals, die Verfahrensstufen a) bis e) gemäß f) wiederholt werden.

Die Ausgangsstoffe (II) sind Monoalkylether von Alkylenglykolen und deren Kondensationsprodukten, den Oligoglykolen,welche ihrerseits durch Sauerstoffbrücken verbundene homogene Ether der genannten Alkylenglykole sind. Ausgangsstoffe sind beispielsweise 1-Methoxipropanol-2; 2-Methoxipropanol-1; Dipropylenglykolmonoethyl-, -n-propyl-, -iso-propyl- oder die isomeren -butylether; 1-Ethoxipropanol-2; 1-n- oder iso-Propoxipropanol-2; 1-sek.-Butoxipropanol-2; Tripropylenglykol-monomethylether; Methyl-, Ethyl-, n- und iso-Propyl-, sec.-oder tertiär-Butyl-monoglykol, oder -diglykol oder -triglykol; Tetraethylenglykol-monomethylether, Penta- oder Hexaethylenglykol-monomethylether oder die entsprechenden Monoether der Butylen- und Dibutylenglykole.

Diesen Ausgangsstoffen ist gemeinsam, daß sie an dem einen Kettenende der Monoglykole oder Polyglykole bereits durch einen niederen Alkylrest verethert sind. Verfahrensgemäß werden sie in das Alkoholat der freien OH-Gruppe umgewandelt durch Umsetzung mit Alkalimetallen, bevorzugt Natrium oder Kalium bzw. Erdalkalimetallen wie Kalzium, oder deren Hydriden, Oxiden, Alkoholaten, festen oder in Wasser gelösten Alkalihydroxiden. Alkoholate, besonders Methylate bzw. Ethylate und Hydroxide des Natriums oder Kaliums sind bevorzugt. Als Fluchtgruppe treten im Falle der Alkalimetalle und Hydride Wasserstoff, im Falle der Alkoholate Alkohole und im Falle Hydroxide Wasser auf.Die in ihr Alkoholat umgesetzten Glykolmonoalkylether (II) werden mit Alkenylhalo-geniden der Formel (III) umgesetzt, beispielsweise mit Allylchlorid, Allylbromid, Allyljodid, Methallylchlorid, Vinylchlorid oder -bromid, 4-Chlorbuten-1, 5-Chlorpenten-1 oder 6-Chlor-, Brom- oder -Jod-hexen-1. Bevorzugte Alkenylhalogenide sind Allylchlorid, Methallylchlorid, Vinylchlorid oder den entsprechenden Bromiden.

Die nach dem erfindungsgemäßen Verfahren hergestellten unsymmetrischen, endständig ungesättigten Glykolether der Formel (I) sind beispielsweise 1-Methoxi-, -Ethoxi-, -n- oder -iso-Propoxi-, -n-, -iso-, -sekundär- oder -tertiär-Butoxipropyl-2-vinyl-, -allyl- und -methallylether; 2-Methoxi-, -Ethoxi, -n- oder -iso-Propoxi-, -n-, -iso-, -sekundär- und -tertiär-Butoxipropyl-vinyl- bzw. -allyl, bzw. -methallyl-1 -butenylether, -pentenylether oder -hexenylether; Dipropylenglykol-monomethyl-monovinyl- oder -allyl- oder -methallyl bzw. -1-butenylether; Tripropylenglykol-monomethyl-monovinyl- oder -allyl- oder -methallylether; Mono-, Di-, Tri-, Tetra-, Penta- und Hexaethylenglykol-monomethyl-, -ethyl-, -n-oder -iso-propyl, -n-, -iso-, -sekundär- oder tertiärbutylmonovinyl, -allyl-, -methallyl-, -1-butenyl-, -pentenyl- oder hexenylether oder die Butylen- und Dibutylen-monoalkylmonovinylether bzw. -methallylether.
Bevorzugte Ausgangsstoffe (II) sind die Monoalkylether der Oligoalkylenglykole, nämlich von Diethylenglykol, Triethylenglykol und Tetraethylenglykol sowie von monomerem Ethylenglykol. Das Verfahren wird vorzugsweise in einem Reaktor ausgeführt, der mit einem Rührwerk ausgestattet ist, das die Zugabe von Suspensionen oder rieselfähigen Feststoffen erlaubt. Der Reaktor kann mit Rückflußkühler mit Destillatabnahme- Einrichtung, Brüdenverdichtung, Vakuumanschlüssen und regelbarer Beheizung ausgerüstet werden. Um Sauerstoff auszuschließen, ist eine Überdeckung mit Stickstoff vorgesehen. Während der Entfernung der Fluchtgruppe ist ständig der Siedezustand, ggf. unter vermindertem Druck, einzuhalten.

Während der Bindung der überschüssigen Ausgangsstoffe (II) als oder an das Alkoholat vor der destillativen Abtrennung der Produkte (I) aus dem Reaktionsgemisch und während der Destillation der Produkte (I) ist eine Begrenzung der Temperatur über alle Verfahrensmaßnahmen auf max. 130°C, vorzugsweise auf 110°C einzuhalten.

Die erfindungsgemäße Begrenzung der Temperatur ist ein wirksamer Schutz gegen unerwünschte Isomerisierung der entstandenen Allyl-, 1-Butenyl- bzw. Methallylgruppe der Produkte in die cis-trans-Propyl-, Crotonyl bzw. die Isobutenylether. Wir fanden, daß diese Umlagerung thermisch ausgelöst wird. Diese Temperaturbegrenzung erfordert Steuerungsmaßnahmen für die Reaktionsbedingung des ständigen Siedens bei der Durchführung der Alkoholat-Stufe. Die Steuerung erfolgt durch die Anwendung von auf das Siedeverhalten des Reaktionsgemisches abgestimmtem Unterdruck und/oder mit Hilfe eines zur Siedepunkteinstellung geeigneten inerten Lösungsmittels aus der Gruppe der Kohlenwasserstoffe, z.B. Toluol, Isooctan, die Xylol-Isomeren, wie Mesitylen, Aromatenbenzine mit definierten Siedepunkten etc. Die Lösungsmittel können gleichzeitig als Schleppmittel für die Fluchtgruppe und als Verdünner bei der Abtrennung der Metallhalogenide durch Filtration dienen.

Bei Beginn der Reaktionen wird der Ausgangsstoff (II) bevorzugt zusammen mit dem Alkoholatbildner (2:1 Äquivalente) vorgelegt.

Die Siedebedingung wird bevorzugt mit Hilfe von Unterdruck und/oder einem Lösungsmittel eingestellt. Die Fluchtgruppe muß vollständig ausdestilliert werden.

Das flüssige abfiltrierte oder abdestillierte Gemisch von (I) und (II) wird in den Reaktor zurückgeführt.
Vor der Gewinnung des Produktes (I) braucht keine vollständige Umsetzung des Alkoholatbildners zu erfolgen; diese wird nach der Gewinnung der Produkte vervollständigt.
Nach der Gewinnung der Produkte kann das Verfahren durch Neutralisation des im Reaktor zurückgebliebenen Alkoholats abgebrochen werden.

Die Verwendung des ungestättigten Glykolethers der Formel (I) erfolgt durch Umsetzung zu Silanen durch Anlagerung von Hydrogenalkoxysilanen.
Es entstehen Produkte der Formel 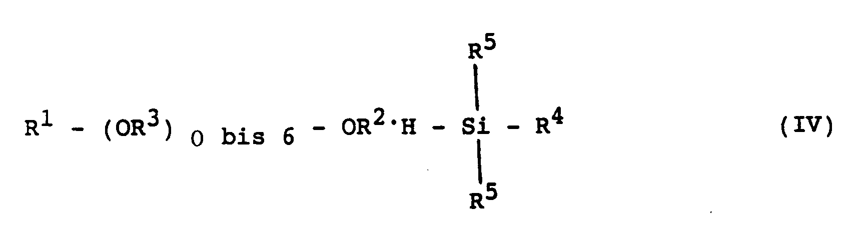
mit R¹, R² und R³ in der genannten Bedeutung, R⁴ in der Bedeutung R¹ - O oder R¹ -(OR³) _{0 bis 6} - O - und R⁵ in der Bedeutung R¹ oder R⁴. Diese Produkte sind Komponenten von Hydraulikflüssigkeit, die aus der DE-PS 2 652 719 bekannt sind, entstehen aber erst durch die Hydrosilierung von Stoffen der Formel (I) in brauchbarer Reinheit.

### Beispiel 1

### 1-Methoxy-2-methallyloxypropan

### A) Synthese:

In einem 4l-Zylindergefäß mit einem mit randgängigem Strömungsbrecher ausgerüstetem Feststoffrührwerk, versehen mit Thermostatbeheiztem Doppelmantel, 6-Boden-Füllkörperkolonne, N₂-Überdeckung, Destillatvorlage und Vakuumerzeugung, wurden 1803 g (20 Mol) 1-Methoxypropanol-2 und 540 g (10 Mol) festes Natriummethylat vorgelegt. Beim Aufheizen auf 124°C destillierten innerhalb von 60 Minuten 220 g Methanol ab, wobei das Natriummethylat vollständig in Lösung ging. Weitere 74 g Methanol wurden unter gleichen Bedingungen innerhalb einer weiteren Stunde über eine Kolonne abdestilliert. Danach wurde die Destillatabnahme geschlossen, der Ansatz auf 60° abgekühlt und bei dieser Temperatur bei 50 mbar Unterdruck unter Rückfluß 1 Std. weiter gekocht, wobei restliche 21 g Methanol über den Rückflußkühler aus dem Ansatz entwichen und in der bei -80°C arbeitenden Kühlfalle der Vakuumerzeugung aufgefangen wurden. Nachdem sich im Ansatz und im Rückflußkondensat kein Methanol mehr nachweisen ließ, wurden innerhalb 100 Minuten unter Rühren und Thermostatenkühlung von 60°C 906 g (10 Mol) Metallylchlorid in den Reaktor dosiert. Die exotherme Reaktion erhöhte die Temperatur im Reaktor auf 104°C. Dabei fiel Natriumchlorid aus. Es wurde 70 Minuten lang bei 110°C nachgerührt. Danach wurde die Kolonne gegen einen Destillationsaufsatz für Kurzwegdestillation ausgetauscht und das aus fast genau je 10 Mol 1-Methoxipropanol-2 und 1-Methoxi-2-methallyloxipropan bestehende Stoffgemisch erst bei 90°C/100 mbar, dann bei auf 10 mbar abgesenktem Druck bei fallendem Siedepunkt vom NaCl-Niederschlag abdestilliert und das NaCl schließlich bei 1 mbar ausgetrocknet, wobei das restliche Produkt in der Kühlfalle aufgefangen wurde.

Insgesamt wurden 2336 g Destillat erhalten mit der Zusammensetzung von 62 Gew.-% 1-Methoxi-2-methallyloxipropan und 39 Gew.-% 1-Methoxipropanol-2. Aus dem Reaktor wurden 580 g trockenes, rieselfähiges NaCl entleert.

### B) Isolierung:

Der Reaktor gemäß 1 A) wurde mit den 2336 g Enddestillat der Verfahrensmaßnahme A) und mit 540 g (10 Mol) festem Natriummethylat beschickt. Beim Aufheizen auf 124°C destillierten innerhalb von 60 Minuten 220 g Methanol ab, wobei das Natriummethylat in Lösung ging. Bei der Destillation bei 90°C/100 mbar gingen noch 29 g Methanol über. Dann versiegte die Methanol-Abspaltung. Auch das Kochen des Ansatzes bei 70°C/20 mbar brachte Methanol nur noch in Spuren in die Kühlfalle. Danach wurde die Kolonne gegen einen Destillationsaufsatz für Kurzwegdestillation ausgetauscht und der Ansatz beim Siedepunkt von 70°C/20 mbar ausdestilliert bis bei einer wieder bis 124°C ansteigenden Reaktortemperatur nichts mehr überging. Es wurden dabei 1368 g 1-Methoxi-2-methallyloxipropan als reines Produkt erhalten. Das entspricht einer Reinausbeute von 94-95%, bezogen auf je 10 Mol Ausgangsstoffe.

| | |
|---|---|
| Kp. | 96°C/122 mbar |
| D.₄ ²⁰ | 0.861 |
| Viskosität (20°C) | 0.77 mPa·s |

### C) Synthese-Cyclus:

Der Reaktor enthält den Rückstand der Destillation, im wesentlichen 10 Mol (II), überwiegend umgesetzt mit Natriummethylat. Der Kurzwegdestillationsaufsatz wird durch einen Rückflußkühler ersetzt. 10 Mol (902 g) 1-Methoxypropanol-2 wurden zugesetzt, so daß die Stoffmenge des Schrittes 1 A) vorlag. Bei 60°C/50 mbar wurde 2 Stunden unter Rückfluß gekocht, wobei noch 54 g Methanol in der Kühlfalle erfasst wurden. Dann wurde weiter analog 1 A) mit Methallylchlorid umgesetzt und aufgearbeitet. Es wurden 2382 g Destillat erhalten mit der Zusammensetzung von 64 Gew.-% Produkt und 36 Gew.-% 1-Methoxypropanol-2. Daraus wurden durch Fortsetzung des Verfahrens analog Verfahrensmaßnahme B) 1414 g Reinprodukt entsprechend einer Reinausbeute von 98% isoliert.

Der Synthese-Cyclus gemäß 1 C) beschreibt das Chargen-Verfahren für die zu wiederholende Herstellung von 1-Methoxy-2-Metallyloxipropan.

### Beispiel 2

### Diethylenglykol-monomethyl-monoallylether

### A) Synthese:

Im Reaktionsgefäß gemäß Beispiel 1, jedoch mit wassergekühltem Rückflußkühler (0,4 m² Kühlfläche), N₂-Überdeckung und 1l-Kühlfalle (-80°C) vor der Vakuumerzeugung, wurden 2020 g (16 Mol) Diethylenglykolmonomethylether (MDG) und 449g (8 Mol) festes Kaliumhydroxid vorgelegt und der Druck auf 5 mbar vermindert. Unter Wärmezufuhr begann das Reaktionsgemisch bei 44° C zu sieden und Wasser abzuspalten, das unter MDG-Rückfluß in die Kühlfalle gezogen (Kühlerabgangstemperatur 17°C) und dort ausgefroren wurde. Nach 200 Minuten war eine Siedetemperatur von 82°C erreicht und das Kaliumhydroxid hatte sich vollständig gelöst. Nach weiteren 180 Minuten bei 82 bis 86°C Reaktortemperatur war die Abspaltung von Wasser abgeschlossen. Insgesamt wurden 147 g Wasser abgespalten. Anschließend wurden unter Normaldruck und Thermostantenkühlung bei 50° innerhalb von 120 Minuten 988 g (8 Mol) Allylbromid in den Reator dosiert. Unter exothermer Reaktion stieg die Reaktortemperatur bis auf 89°C. Dabei fiel Kaliumbromid aus. Es wurde 60 Minuten lang bei 90°C nachgerührt. Dann wurde der Rückflußkühler gegen einen Destillationsaufsatz für Kurzwegdestillation ausgetauscht und das aus je 8 Mol MDG und Diethylenglykol-monomethyl-monoallylether bestehende Produktgemisch bei 42 bis 44°C/ 2 mbar vom KBr-Niederschlag vollständig abdestilliert. Insgesamt wurden 2202 g Destillat mit der Zusammensetzung von 55% Produkt und 45% MDG erhalten. Aus dem Reaktor wurden 959 g trockenes, rieselfähiges KBr entleert.

### B) Isolierung:

Der Reaktor gemäß 2 A) wurde mit den 2202 g Enddestillat der Verfahrensmaßnahme 2 A) und 449 g (8 Mol) festem Kaliumhydroxid beschickt. Analog 2 A) wurden innerhalb 280 Minuten 109 g Wasser abgespalten. Nach weiteren 2 Std. fand trotz unvollständiger Umsetzung keine merkliche Wasserabspaltung mehr statt. Insgesamt wurden nur 126 g Wasser erhalten, d.h. 88% der zu erwartenden Wassermenge. Daraufhin wurde der Rückflußkühler gegen einen Destillationsaufsatz für Kurzwegdestillation ausgetauscht und aus dem Ansatz 1020 g Diethylenglykol-monomethyl-monoallylether in reiner Form abdestilliert. Das sind 86% der zu erwartenden Ausbeute. Die restlichen 14% wurden aus praktischen Gründen im Ansatz belassen zur Unterstützung der flüssigen Konsistenz. Sie wurden im anschließenden Synthese-Cyclus gewonnen.

| | |
|---|---|
| Kp. | 45°C/ 2 mbar |
| D.₄²⁰ | 0.941 |
| Viskosität (20°C) | 1.41 mPa·s |

### C) Synthese-Cyclus:

Der Reaktorinhalt nach der Enddestillation der Verfahrensmaßnahme 2 B) wurde nach Austausch des Destillationsaufsatzes gegen den Rückflußkühler gemäß 2 A) durch Zugabe von neuen 8 Mol (1010 g) MDG auf die Stoffeinsatzzahl des Syntheseschrittes gemäß 2 A) ergänzt und daraus in 2 Std. unter Rückfluß bei 83°C und 5 mbar restliche 19 g Wasser abgespalten. Dann wurde weiter analog 2 A) mit Allylbromid umgesetzt und aufgearbeitet. Es wurden 2400 g Destillat erhalten mit der Zusammensetzung von 58 Gew.-% Produkt und 42 Gew.-% MDG. Daraus wurden durch die Fortsetzung des Verfahrens analog der Verfahrensmaßnahme 2 B) 1157 g reines Produkt entsprechend einer Reinausbeute von 96% isoliert.

Der Synthese-Cyclus gemäß 2 C) beschreibt das Chargen-Verfahren für die zu wiederholende Herstellung von Diethylenglykolmonomethyl-monoallylether.

### Beispiel 3

### Triethylenglykol-monomethyl-monoallylether

### A) Synthese:

Analog Beispiel 2 A) wurden 1970 g (12 Mol) Triethylenglykolmonomethylether (MTG) und 240 g (6 Mol) festes Natriumhydroxid bei 2 mbar unter Beheizung auf 110°C und Wasserabspaltung ins Alkoholat überführt. Die Wasserabspaltung setzte bei 40°C ein und war nach dem Austrag von 110 g Wasser nach 6 Std. beendet. Anschließend wurden bei 70°C Steuertemperatur innerhalb von 50 Minuten 460 g (6 mol) Allylchlorid eindosiert, wobei die Reaktortemperatur auf 90°C anstieg und Natriumchlorid ausfiel. Die Kurzwegdestillation bei 90-92° C/ 2 mbar lieferte 2191 g äquimolares Gemisch bestehend aus 44% Methyltriglykol und 56% Triethylenglykol-monomethyl-monoallylether. Der Destillationsrückstand bestand aus 362 g trockenem, rieselfähigen NaCl.

### B) Isolierung:

Analog Beispiel 2 B) wurden aus den 2191 g Enddestillat der Verfahrensmaßnahme 3 A) und 240 g (6 Mol) festem NaOH innerhalb 330 Minuten 91 g Wasser abgespalten, d.h. 84 % der zu erwartenden Wassermenge. Dann wurden aus dem Ansatz 942 g reiner Triethylenglykol-monomethyl-monoallyether abdestiliert (Kp. 84°C/1 mbar). Das sind 77% der zu erwartenden Ausbeute. Die restlichen 23% der Ausbeute wurden zur Erhaltung einer besser handhabbaren flüssigen Konsistenz im Ansatz belassen. Sie wurden im anschließenden Synthese-Cyclus gewonnen.

| | |
|---|---|
| Kp. | 84°C/ 1 mbar |
| D·₄²⁰ | 0.975 |
| Viskosität (20°C) | 2.69 mPa·s |

### C) Synthese-Cyclus:

Der Reaktorinhalt wurde analog Beilspiel 2 C) nach Zugabe weiterer 6 Mol (985 g) MTG bei 110°C / 2 mbar in 160 Minuten vom restlichen Wasser (20 g) befreit und damit die Alkoholat-Herstellung abgeschlossen, analog 3 A) mit 460 g (6 Mol) Allylchlorid umgesetzt und aufgearbeitet. Es wurden 2478 g Destillat erhalten mit der Zusammensetzung von 61 Gew.-% Produkt und 39 Gew.-% MTG. Daraus wurden durch die Fortsetzung des Verfahrens analog der Verfahrensmaßnahme 3 B) 1146 g Reinprodukt entsprechend einer Reinausbeute von ca. 94% isoliert.

Der Synthese-Cyclus gemäß 3 C) beschreibt das Chargen-Verfahren für die zu wiederholende Herstellung von Triethylenglykolmonomethyl-monoallylether.

### Beispiel 4

### Triethylenglykol-monomethyl-monomethallylether

### A) Synthese:

An der Apparatur gemäß Beispiel 2 A) wurde an den Rückflußkühler die Saugseite einer Wälzkolbenpumpe (Saugleistung 30 m³/h) angeschlossen, deren Druckseite über einen Kühlwasser-betriebenen Brüdenkondensator (0,4 m² Austauschfläche) mit einer auf 35 mbar eingestellten Vorvakuumpumpe verbunden war.

Die Apparatur wurde mit 1970 g (12 Mol) Triethylenglykolmonomethylether (MTG) und 480 g (6 Mol) 50 Gew.-%ig wäßriger Natronlauge beschickt.
Innerhalb von ca. 260 Minuten wurden mit Siedebeginn bei 38°C bei langsam auf 82° gesteigerter Temperatur 320 g Wasser abgezogen; innerhalb weiterer 100 Minuten bei weiter auf 110°C gesteigerter Temperatur weitere 22 g Wasser; und schließlich innerhalb weiterer 40 Minuten weitere 10 g Wasser unter Beibehaltung von 110°C. Die gesamte Wasserabspaltung betrug 352 g. Analog Beispiel 2 A) wurde anschließend bei 80°C Steuertemperatur innerhalb von 80 Minuten mit 543 g (6 Mol) Methallylchlorid umgesetzt. Die Kurzwegdestillation bei 90 -92°C / 2 mbar lieferte 2283 g äquimolares Gemisch von 57% Triethylenglykol-monomethyl-monomethallylether und 43% MTG und 365 g NaCl.

### B) Isolierung:

Analog Beispiel 2 B), jedoch mit der Vakuumeinrichtung gemäß 4 A) wurden aus dem vorstehenden Destillat und 240 g (6 Mol) festem NaOH innerhalb von 28 Minuten 93 g Wasser abgespalten, d.h. 86% der zu erwartenden Wassermenge. Dann wurden aus dem Ansatz 969 g reiner Triethylenglkol-monomethyl-monomethallyether abdestilliert (Kp. 89°C / 1 mbar). Das sind 74% der Produktausbeute. Die restlichen 26% der zu erwartenden Ausbeute wurden zur Erhaltung flüssiger Konsistenz im Ansatz belassen. Sie wurden im anschließenden Synthese-Cyclus gewonnen.

| | |
|---|---|
| Kp. | 89°C / 1 mbar |
| D·₄²⁰ | 0.965 |
| Viskosität (20°C) | 2.94 mPa·s |

### C) Synthese-Cyclus:

Der Reaktorinhalt nach der Verfahrensmaßnahme 4 B) wurde analog 4 A) nach Zugabe weiterer 6 Mol (985 g) MTG in 17 Minuten vom restlichen Wasser (19 g) befreit, wieder mit 543 g ( 6 Mol) Methallylchlorid umgesetzt und aufgearbeitet. Es wurden als Destillat 2612 g äquimolares Gemisch von 59 Gew.-% Produkt und 41 Gew.-% Methyltriglykol erhalten. Daraus wurden durch Fortsetzung des Verfahrens analog der Verfahrensmaßnahme 4 B) 1230 g Reinprodukt entsprechend einer Reinausbeute von 94% isoliert.
Der Cyclus 4 C) kann, wie in den vorangehenden Beispielen, wiederholt werden.

### Beispiel 5

### Triethylenglykol-monobutyl-monoallylether

### A) Synthese:

Analog Beispiel 2A) wurden 2063 g (10 Mol) Triethylenglykolmonobutylether (BTG) und 200 g (5 Mol) festes Natriumhydroxid innerhalb 5 Stunden bei 2 mbar und 115°C unter Abspaltung von 93 g Wasser ins Alkoholat überführt und anschließend mit 383 g (5 Mol) Allylchlorid gemäß 3 A), umgesetzt. Die Destillation lieferte 2243 g Gemisch aus 55% Produkt und 45% BTG neben 300 g NaCl.

### B) Isolierung:

Analog Beispiel 2 B) wurde aus vorstehendem Destillat und 200 g NaOH innerhalb von 220 Minuten 70 g Wasser (78% der bei vollständiger Reaktion zu erwartenden Menge) abgespalten. Dann wurden aus dem Ansatz 841 g reiner Triethylenglykol-monobutylmonoallylether abdestilliert. Das sind ca. 68% der Gesamtausbeute. Die restliche Ausbeute wurde im Synthese-Cyclus C) isoliert.

| | |
|---|---|
| Kp. | 92° C / 1 mbar |
| D·₄²⁰ | 0.941 |
| Viskosität (20° C) | 3.56 mPa.s |

### C) Synthese-Cyclus:

Der Reaktorinhalt nach 5 B) wurde analog Beispiel 2 C) nach Zugabe weiterer 5 Mol (1032 g) BTG bei 115°C / 2 mbar in 150 Minuten vom restlichen Wasser (20 g) befreit und wieder mit 5 Mol (383 g) Allylchlorid gemäß 5 A) umgesetzt und aufgearbeitet. Es wurden ca. 2640 g Destillat erhalten (Gemisch aus 61 Gew.-% Produkt und 39 Gew.-% BTG). Daraus wurden durch die Fortsetzung des Verfahrens analog der Verfahrensmaßnahme 5 B) 1114 g Triethylenglykol-monobutyl-monoallylether entsprechend einer Reinausbeute von 90% isoliert.
Der Cyclus 5 C) wurde wiederholt.

### Beispiel 6

### Tetraethylenglykol-monomethyl-monoallylether

### A) Synthese:

Analog Beispiel 2 A) wurden 2083 g (10 Mol) Tetraethylenglykol-monomethylether (MTeG) und 200 g (5 Mol) NaOH innerhalb ca. 6 Stunden bei 2 mbar und 118°C unter Abspaltung von 92 g Wasser ins Alkoholat überführt und anschließend mit 383 g (5 Mol) Allylchlorid umgesetzt. Die Destillation lieferte 2266 g Gemisch aus 45% MTG und 55% Tetraethylenglykol-monomethyl-monoallylether neben 300 g NaCl.

### B) Isolierung:

Analog Beispiel 2 B) wurde aus vorstehendem Destillat und 200 g NaOH innerhalb von 240 Minuten 75 g Wasser (ca. 83% der bei vollständiger Reaktion zu erwartenden Menge) abgespalten. Dann wurden aus dem Ansatz 868 g reiner Tetraethylenglykol-monomethyl-monoallylether abdestilliert. Das sind 70% der insgesamt zu erwartenden Ausbeute. Die restlichen 30% wurden im anschließenden Synthese-Cyclus gewonnen.

| | |
|---|---|
| Kp. | 98°C/1 mbar |
| D·₄²⁰ | 1.003 |
| Viskosität (20°C) | 4.49 mPa.s |

### C) Synthese-Cyclus:

Der Reaktorinhalt nach 6 B) wurde analog Beispiel 2 C) nach Zugabe weiterer 5 Mol (1042 g) Methyltetraglykol bei 118°C/ 1 mbar in 140 Minuten vom restlichen Wasser (17 g) befreit und wieder mit 5 Mol (383 g) Allylchlorid gemäß 6 A) umgesetzt und aufgearbeitet. Es wurden 2635 g Destillat erhalten (Gemisch aus 61 Gew.-% Produkt und 39 Gew.-% MTeG). Daraus wurden durch die Fortsetzung des Verfahrens analog der Verfahrensmaßnahme 6 B) 1127 g Reinprodukt entsprechend einer Reinausbeute von 92% isoliert.
Der Cyclus 6 C wurde wiederholt.

### Beispiel 7

### Tetraethylenglykol-monoethyl-monoallylether

### A) Synthese

Analog Beispiel 6 wurden 1780 g (8 Mol) Tetraethylenglykolmonomethylether (ETeG) und 160 g (4 Mol) festes NaOH bei 1 mbar und 114°C unter Abspaltung von 73 g Wasser ins Alkoholat überführt und mit 306 g (4 Mol) Allylchlorid, analog 6 A), umgesetzt. Die Destillation lieferte 1924 g Gemisch aus 46% ETG und 54% Tetraethylenglykol-monoethyl-monoallylether neben 240 g NaCl.

### B) Isolierung:

Mit 160 g (4 Mol) NaOH und vorstehendem Destillat wurden 59 g Wasser (81 % der Gesamtmenge) erhalten. Dann wurden aus dem Ansatz 735 g reiner Tetraethylenglykol-monoethylmonoallylether abdestilliert. Das sind 70% der insgesamt zu erwartenden Ausbeute. Die restliche Ausbeute wurde im anschließenden Cyclus 7 C) gewonnen.

| | |
|---|---|
| Kp. | 106°C / 0.5 mbar |
| D.₄²⁰ | 0.971 |
| Viskosität (20° C) | 4.41 mPa.s |

### C) Synthese-Cyclus:

Weiter analog Beispiel 6 wurde nach Zugabe von 4 Mol (890 g) ETeG das restliche Wasser (ca. 15 g) abgeführt und mit 4 Mol (306 g) Allylchlorid umgesetzt. Die Aufarbeitung ergab 2224 g Destillat (mit 61 Gew.-% Produkt neben Ethyltriglykol). Daraus wurden durch die Fortsetzung des Verfahrens analog der Verfahrensmaßnahme 7 B) 964 g Tetraethylenglykol-monoethyl-monoallylether isoliert, was einer Gesamtausbeute von 92% entspricht.
Der Synthese-Cyclus gemäß 7 C) wurde wiederholt.

### Beispiel 8

### Pentaethylenglykol-monomethyl-monoallylether

### A) Synthese:

Analog Beispiel 6 wurden 1514 g (6 Mol) Pentaethylenglykolmonomethylether (MPG) und 120 g (3 Mol) festes Natriumhydroxid bei 1 mbar und 120° g unter Abspaltung von 56 g Wasser ins Alkoholat überführt und mit 230 g (3 Mol) Allylchlorid umgesetzt. Die Destillation lieferte 1620 g Gemisch (54% Pentaethylenglykol-monomethyl-monoallylether neben Methylpentaglykol) und 180 g NaCl.

### B) Isolierung:

Aus vorstehendem Destillat und 120 g (3 Mol) NaOH wurden 43 g Wasser (80% der Gesamtmenge) abgespalten. Dann wurden aus dem Ansatz 571 g reiner Pentaethylenglykol-monoethyl-monoallylether abdestilliert. Das sind 65% der Gesamtausbeute. Die restlichen Anteile wurden im Synthese-Cyclus 8 C) gewonnen.

| | |
|---|---|
| Kp. | 114°C / 0.6 mbar |
| D·₄²⁰ | 1.020 |
| Viskosität (20° C) | 6.35 mPa.s |

### C) Synthese-Cyclus:

Weiter analog Beispiel 6 wurde nach Zugabe von 3 Mol (757 g) MPG zum Reaktorinhalt das restliche Wasser (ca. 12 g) abgespalten und mit 3 Mol (230 g) Allylchlorid umgesetzt. Die Aufarbeitung ergab 1919 g Destillat (mit 61 Gew.-% Produkt neben Methylpentaglykol). Daraus wurden durch die Fortsetzung des Verfahrens analog der Verfahrensmaßnahme 8 B) 797 g Pentaethylenglykolmonomethyl-monoallylether isoliert, was einer Gesamtausbeute von 91% entspricht.
Der Cyclus 8 C) wurde wiederholt.

### Beispiel 9

### Tetraethylenglykol-monobutyl-monoallylether

Analog Beispiel 6 wurde aus Butyltetraglykol, Natriumhydroxid und Allylchlorid in 94% Ausbeute Tetraethylenglykol-monobutyl-monoallylether hergestellt.

| | |
|---|---|
| Kp. | 122°C/1 mbar |
| D.₄²⁰ | 0.966 |
| Viskosität (20°C) | 5.48 mPa.s |

### Beispiel 10

### Triethylenglykol-monomethyl-monovinylether

Analog Beispiel 1 wurde in einem 1 l-Apparat in 2 molaren Ansätzen aus Methyltriglykol, Natriummethylat und Vinylbromid unter Ausscheidung von NaBr das Zielprodukt hergestellt.

| | |
|---|---|
| Kp. | 76°C / 1 mbar |
| D.₄²⁰ | 1.047 |
| Viskosität (20°C) | 6.85 mPa·s |

## Patentansprüche

1. Verfahren zur Herstellung unsymmetrischer, endständig einfach ungesättigter Glykolether der Formel
R¹ - (OR³) _{1 bis 6} - OR² (I),
worin R³ gleiche oder verschiedene Alkylengruppen von 2 bis 4 C-Atomen in der Kette mit ggf. an die C-Atome gebundenen Methylgruppen, R¹ einen geradkettigen oder verzweigten Alkylrest mit 1 bis 4 C-Atomen und R² einen endständig ungesättigten Alkenylrest mit 2 bis 6 C-Atomen in der Kette mit ggf. an die C-Atome gebundenen Methyl- oder Ethylgruppen bedeuten, durch Überführung von Monoalkylethern der Formel
R¹ - (O R³) _{1 bis 6} - OH (II),
mit Alkalimetallen bzw. Erdakalimetallen, deren Alkoholaten bzw. Oxiden, Hydriden oder Hydroxiden in die Alkoholate und nachfolgender Umsetzung der Alkoholate mit einem endständig ungesättigten Alkenylhalogenid oder Methallylhalogenid oder Vinylhalogenid der Formel
X - R² (III),
worin X Chlorid, Bromid oder Jodid bedeuten und R² die genannte Bedeutung hat , dadurch gekennzeichnet, daß
a) man zunächst zwei Äquivalente (II) mit einem Äquivalent der alkoholatbildenden Metallkomponente bis zu deren vollständigem Umsatz unter Sauerstoff-Ausschluß umsetzt, wobei die jeweilige Fluchtgruppe abdestilliert wird,
b) das entstandene (II)-Alkoholat mit einem Äquivalent (III) ausreagieren läßt,
c) das erhaltene Gemisch der Stoffe (I) und (II) vom ausgefällten Metallhalogenid abfiltriert oder vollständig abdestilliert,
d) das Destillat bzw. Filtrat mit einem zweiten Äquivalent des Alkoholat-Bildners unter den Bedingungen von a) umsetzt,
e) danach das Produkt (I) weitgehend abdestilliert und
f) dem nach der Gewinnung von (I) zurückbleibenden alkoholathaltigen Reaktionsgemisch ein weiteres Äquivalent (II) zusetzt, die Umsetzung gemäß a) zu Ende führt und wieder die Verfahrensschritte a) bis e) ausführt.

2. Verfahren nach Anspruch 1, **dadurch gekennzeichnet,** daß die Verfahrenstemperatur ständig auf maximal 130° C begrenzt ist.

3. Verfahren nach Anspruch 1, **dadurch gekennzeichnet,** daß die Verfahrentemperatur auf 110° C begrenzt ist.

4. Verfahren nach einem der Ansprüche 1 bis 3, **dadurch gekennzeichnet,** daß der Verfahrensschritt a) bei Siedetemperatur erfolgt.

## Claims

1. Process for the production of asymmetrical terminally singly unsaturated glycol ethers of the Formula
R¹ - (OR³) _{1 to 6} - OR₂ (I),
wherein R³ denotes like or different alkylene groups of 2 to 4 C-atoms in the chain with possibly methyl groups bound to the C-atoms, R¹ denotes a straight-chain or branched alkyl residue with 1 to 4 C-atoms and R² denotes a terminally unsaturated alkenyl residue with 2 to 6 C-atoms in the chain with possibly methyl or ethyl groups bound to the C-atoms, by conversion of monoalkylethers of the formula
R¹ - (O R³) _{1 to 6} - OH (II),
by means of alkali metals or alkaline earth metals, alcoholates or oxides thereof, hydrides or hydroxides into the alcoholates and subsequent reaction of the alcoholates with a terminally unsaturated alkenyl halide or methallyl halide or vinyl halide of the Formula
X -R² (III),
wherein X denotes chloride, bromide or iodide and R² has the indicated meaning, characterised in that
a) initially two equivalents of (II) are reacted with one equivalent of the alcoholate forming metal components until its complete conversion, with exclusion of oxygen, wherein the respective labile group is distilled off,
b) the (II)-alcoholate being produced is reacted fully with one equivalent of (III),
c) the mixture obtained of substances (I) and (II) is filtered off from precipitated metal halide or is distilled off completely,
d) the distillate or filtrate is reacted with a second equivalent of the alcoholate former under the conditions of a),
e) then the product (I) is distilled off to a large extent and
f) a further equivalent of (II) is added to the alcoholate containing reaction mixture remaining behind after the recovery of (I), the reaction according to a) is taken to completion and the process steps a) to e) are carried out again.

2. Process according to claim 1, characterised in that the process temperature is limited at all times to a maximum of 130°C.

3. Process according to claim 1, characterised in that the process temperature is limited to 110°C.

4. Process according to one of claims 1 to 3, characterised in that the process step a) takes place at boiling temperature.

## Revendications

1. Procédé de préparation des éthers de glycols à mono-insaturation terminale, asymétriques, de formule :
R¹ - (OR³) _{1 à 6} - OR² (I),
dans laquelle R³ représente des groupes alkylène identiques ou différents ayant 2 à 4 atomes de carbone dans la chaîne avec éventuellement des groupes méthyles fixés sur les atomes de carbone, R¹ représente un reste alkyle linéaire ou ramifié ayant 1 à 4 atomes de carbone, et R² représente un reste alcényle à insaturation terminale comportant 2 à 6 atomes de carbone dans la chaîne avec éventuellement des groupes méthyles ou éthyles fixés sur les atomes de carbone par transformation d'éthers monoalkyliques de formule:
R¹ - (OR³) _{1 à 6} - OH (II),
avec des métaux alcalins ou des métaux alcalino-terreux, leurs alcoolates ou leurs oxydes, leurs hydrures ou leurs hydroxydes, en alcoolates, et réaction subséquente des alcoolates avec un halogénure d'alcényle à insaturation terminale ou avec un halogénure de méthallyle ou avec de l'halogénure de vinyle, de formule:
X - R² (III),
(dans laquelle X représente un ion chlorure, bromure ou iodure, et R² a le sens précité), caractérisé en ce que :
(a) on fait réagir tout d'abord deux équivalents de (II) avec un équivalent du constituant métallique formateur d'alcoolate jusqu'à leur réaction complète, à l'abri de l'oxygène , en chassant par distillation les groupes volatils qui apparaissent,
(b) on laisse totalement réagir l'alcoolate (II) ainsi obtenu avec un équivalent de (III),
(c) on sépare par filtration, du mélange obtenu des substances (I) et (II), l'halogénure de métal précipité ou bien on distille complètement ce mélange,
(d) on fait réagir le distillat ou le filtrat avec un second équivalent du formateur d'alcoolate, dans les conditions de (a),
(e) puis l'on chasse de façon poussée, par distillation, le produit (I) et
(f) au mélange réactionnel contenant de l'alcoolate et qui demeure après la récupération de (I), on ajoute un équivalent supplémentaire de (II), on conduit à son terme la réaction selon (a) et l'on exécute à nouveau les étapes opératoires (a) à (e).

2. Procédé selon la revendication 1, caractérisé en ce que la température est constamment limitée à une valeur maximalede 130°C.

3. Procédé selon la revendication 1, caractérisé en ce que la température du procédé est limitée à 110°C.

4. Procédé selon l'une des revendications 1 à 3, caractérisé en ce que l'étape opératoire(a) est effectuée à la température de l'ébullition.
